# EUROPÄISCHE PATENTSCHRIFT

(11) **EP 0 789 744 B1**
(45) Veröffentlichungstag und Bekanntmachung des Hinweises auf die Patenterteilung: **15.12.1999**
(21) Anmeldenummer: 95937827.4
(22) Anmeldetag: 24.10.1995
(51) Int. Cl.: C11D 1/72, C08G 65/32, C07C 41/16, C07C 43/11

(54) **VERFAHREN ZUR HERSTELLUNG VON ENDGRUPPENVERSCHLOSSENEN NICHTIONISCHEN TENSIDEN**
METHOD OF PREPARING NON-IONIC SURFACTANTS HAVING CLOSED END GROUPS
PROCEDE DE FABRICATION DE TENSIOACTIFS NON IONIQUES A GROUPES TERMINAUX FERMES

(30) Priorität: 02.11.1994 DE 4439086
(43) Veröffentlichungstag der Anmeldung: 20.08.1997
(73) Patentinhaber: Henkel Kommanditgesellschaft auf Aktien, 40191 Düsseldorf (DE)
(72) Erfinder: BIGORRA LLOSAS, Joaquim, E-08203 Sabadell (ES); BONASTRE, Nuria, E-080210 Barberà del Vallès (ES); TRIUS OLIVA, Antonio, E-Valldoreix (ES); PI SUBIRANA, Rafael, E-08400 Granollers (ES)
(86) Internationale Anmeldenummer: EP9504156
(87) Internationale Veröffentlichungsnummer: WO9614377

(56) Entgegenhaltungen:
- EP-A- 0 161 537
- EP-A- 0 302 487
- EP-A- 0 427 088
- DE-C- 3 744 525
- NL-A- 6 916 304

## Beschreibung

Die Erfindung betrifft ein Verfahren zur Herstellung von endgruppenverschlossenen nichtionischen Tensiden durch Alkoholatbildung zwischen einem Fettalkoholpolyglycolether und einer festen Base in Gegenwart von Alkali- und/oder Erdalkaliborhydriden, Umsetzung des Alkoholats mit Dimethylsulfat sowie Wasserzugabe unter Phasentrennung.

### Stand der Technik

Für eine Vielzahl technischer Prozesse ist die Anwesenheit von Schaum äußerst unerwünscht. So kommt es z. B. sowohl bei der maschinellen Reinigung von Bier- oder Milchflaschen als auch der Spritzreinigung von Automobilblechen nicht nur auf die reinigende bzw. entfettende Wirkung der eingesetzten oberflächenaktiven Mittel an, die Vermeidung von Schaum, der die Anlagenfunktionen stark beeinträchtigen kann, ist von gleich großer Bedeutung. Dies gilt um so mehr, als in vielen Fällen hochaktive, jedoch auch starkschäumende anionische Tenside eingesetzt werden.

Das Problem der Schaumregulierung ist freilich seit langer Zeit bekannt und dementsprechend sind aus dem Stand der Technik eine Vielzahl von mehr oder weniger überzeugenden Problemlösungen bekannt, die in zwei Gruppen eingeteilt werden können:

Bei der einen handelt es sich um Verfahren unter Zugabe von Entschäumern, bei denen es sich vielfach um paraffinische Kohlenwasserstoffe oder Siliconverbindungen handelt. Für die beschriebenen Anwendungen ist dies jedoch in den meisten Fällen unerwünscht. Bei der zweiten Gruppe von Verfahren handelt es sich um den Einsatz von oberflächenaktiven Mitteln, die selbst schaumarm sind und gegebenenfalls zusätzlich noch über entschäumende Eigenschaften verfügen. In der Regel handelt es sich hierbei um nichtionische Tenside oder tensidähnliche Systeme, wie beispielsweise Fettalkoholpropylenglycolether oder Blockpolymere von Ethylen- und Propylenglycol, die allerdings keine ausreichende biologische Abbaubarkeit aufweisen.

Als besonders effektive schaumarme Tenside haben sich end-gruppenverschlossene Fettalkoholpolyglycolether, sogenannte "Mischether" am Markt etabliert, die beispielsweise von R. Piorr in **Fat.Sci.Technol. 89, 106 (1987)** beschrieben werden. Bei diesen Produkten handelt es sich in der Regel um butylendgruppenverschlossene Niotenside, wie sie beispielsweise aus den Schriften **EP-A 0124815, EP-B 0303928, EP-B 0324340, EP-A 0420802, DE-A 3928600** und **DE-C 4243643** bekannt sind.

Eine Sonderstellung nehmen Methylmischether ein, die einen Methyl-Endgruppenverschluß aufweisen und üblicherweise durch Umsetzung der entsprechenden Fettalkoholpolyglycolether mit Methylhalogeniden [**US 4587365, BASF**] oder Dimethylsulfat hergestellt werden.

Aus der **EP 0302487 B1** (BASF) ist in diesem Zusammenhang ein Ein-Topf-Verfahren zur Herstellung von endgruppenverschlossenen Niotensiden bekannt, bei dem man Fettalkoholpolyglycolether in Gegenwart von wäßrigen Alkalihydroxiden mit Dialkylsulfaten umsetzt, wobei die Reaktion bei einer Temperatur im Bereich von 20 bis 60°C stattfindet und die Konzentration an Alkalihydroxid während der gesamten Dauer der Umsetzung nicht kleiner als 35 Gew.-% - bezogen auf die wäßrige Phase - sein darf. Die Produkte enthalten jedoch bis zu 25 Gew.-% nichtumgesetztes Ausgangsprodukt und sind farblich nicht akzeptabel.

Die EP-A-161 537 offenbart ein Verfahren zur Herstellung von endgruppenverschlossenen Fettalkoholpolyglycolether, wobei man Fettalkoholpolyglycolether bei Raumtemperatur mit einer äquimolaren Menge an Kaliumhydroxid umsetzt, die gebildeten Alkoholate mit Dimethylsulfat verethert und die anorganischen Reaktionsprodukte durch Extraktion mit Wasser vom endgruppenverschlossenen Fettalkoholpolyglycolether abtrennt. Die Produkte sind jedoch in ihrer Farbqualität verbesserungsbedürftig.

Die Aufgabe der Erfindung hat demnach darin bestanden, ein verbessertes Verfahren zur Herstellung von endgruppenverschlossenen nichtionischen Tensiden vom Methylmischether-Typ zur Verfügung zu stellen, die sich durch einen verringerten Gehalt an nichtumgesetztem Ausgangsmaterial und eine verbesserte Farbqualität auszeichnen.

### Beschreibung der Erfindung

Gegenstand der Erfindung ist ein Verfahren zur Herstellung von endgruppenverschlossenen nichtionischen Tensiden der Formel **(I),** 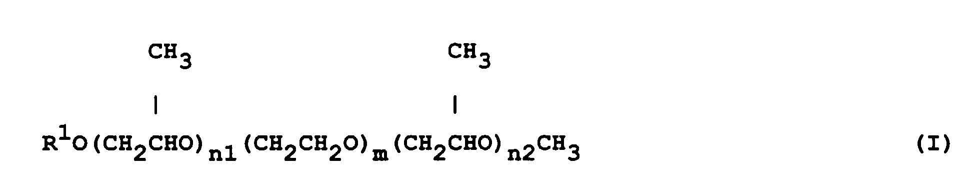 in der R¹ für einen Alkyl- und/oder Alkenylrest mit 6 bis 22 Kohlenstoffatomen, nl und n2 unabhängig voneinander für 0 oder Zahlen von 1 bis 10 und m für Zahlen von 1 bis 20 steht, durch Umsetzung von Alkoholalkoxylaten mit Dimethylsulfat, dadurch gekennzeichnet, daß man
(a) Fettalkoholpolyglycolether der Formel **(II),** 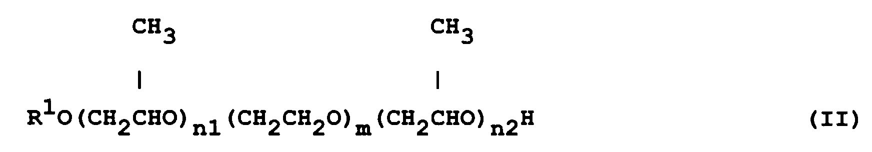 in der R¹, n1, n2 und m die oben angegebenen Bedeutungen besitzen, in Gegenwart von Alkali- und/oder Erdalkaliborhydriden mit festen Basen **(III)** mit einem Wassergehalt von weniger als 15 Gew.-% umsetzt,
(b) die gebildeten Alkoholate mit Dimethylsulfat verethert,
(c) den rohen Ethern eine solche Menge Wasser zusetzt, daß Phasentrennung eintritt und
(d) die organischen Wertphasen in an sich bekannter Weise abtrennt.

Aus dem Stand der Technik ist bereits ein Verfahren bekannt, bei dem Alkoholatbildung und Veretherung in Gegenwart wäßriger Basen durchgeführt wird. Überraschenderweise hat es sich jedoch als weitaus vorteilhafter erwiesen, Alkoholatbildung und Veretherung in zwei Stufen durchzuführen und als Reaktionspartner feste, praktisch wasserfreie Basen einzusetzen, da Produkte mit einem geringeren Gehalt an nichtumgesetzten Ausgangsmaterial erhalten werden können. Der Einsatz von Borhydriden in der Alkoholatbildung führt ferner zu Produkten einer erheblich verbesserten Farbqualität.

### Fettalkoholpolyglycolether

Fettalkoholpolyglycolether stellen bekannte nichtionische Tenside dar, die nach den einschlägigen Verfahren der präparativen organischen Chemie beispielsweise durch Anlagerung von Alkylenoxiden an Fettalkohole erhalten werden können. In Abhängigkeit des Alkoxylierungskatalysators können die Ether eine konventionell breite oder aber eine eingeengte Homologenverteilung aufweisen.

Typische Beispiele für Fettalkoholpolyglycolether, die im Sinne der Erfindung als Ausgangsstoffe in Betracht kommen, sind Anlagerungsprodukte von 5 bis 15 Mol Ethylenoxid und gegebenenfalls 1 Mol Propylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol und Erucylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Besonders bevorzugt sind als Ausgangsstoffe Fettalkoholpolyglycolether der Formel **(II)**, in der R¹ für einen Alkylrest mit 12 bis 18 Kohlenstoffatomen, n1 für 0, m für Zahlen von 5 bis 15 und n2 für 1 bzw. in der R¹ für einen Alkylrest mit 6 bis 10 Kohlenstoffatomen, n1 für 1, m für Zahlen von 5 bis 15 und n2 für 0 steht.

### Basen

Als Basen kommen in erster Linie die Oxide, Hydroxide und Carbonate der Alkali- und/oder Erdalkalimetalle in Betracht. Typische Beispiele sind Lithiumhydroxid, Natriumcarbonat, Natriumhydrogencarbonat, Magnesiumoxid, Magnesiumhydroxid, Calciumoxid und Calciumhydroxid. Bevorzugt ist der Einsatz von Natriumhydroxid und/oder Kaliumhydroxid, vorzugsweise von Kaliumhydroxid. Die Basen gelangen als feste Produkte, also beispielsweise Perlen, Schuppen oder Plätzchen zum Einsatz und weisen einen Wassergehalt von weniger als 15, insbesondere weniger als 10 Gew.-% auf. Ein derartiger Wassergehalt kann im Rahmen des erfindungsgemäßen Verfahrens toleriert werden, obschon wasserfreie Produkte - die jedoch nicht ohne weiteres in technischem Umfang verfügbar sind - grundsätzlich bevorzugt wären.

Es hat sich als vorteilhaft erwiesen, die Fettalkoholpolyglycolether **(II)** und die Basen **(III)** im molaren Verhältnis von 1 : 1,0 bis 1 : 1,5 und vorzugsweise 1 : 1,1 bis 1 : 1,4 einzusetzen.

### Alkoholatbildung

Die Alkoholatbildung findet üblicherweise bei einer Temperatur im Bereich von 20 bis 98°C und vorzugsweise 40 bis 80°C statt. Im Rahmen der Erfindung setzt man den Fettalkoholpolyglycolethern Alkali- und/oder Erdalkaliborhydride zu, was zu praktisch farblosen Produkten führt. Typische Beispiele für geeignete Borhydride sind Kaliumborhydrid und Magnesiumborhydrid und insbesondere Natriumborhydrid. Als Stabilisatoren kommen auch Lithiumalanat und hypophosphorige Säure und deren Alkalisalze in Betracht, die letzteren insbesondere auch in Kombination mit Natriumborhydrid. Üblicherweise werden die Borhydride in Mengen von 100 bis 1000 und insbesondere 300 bis 700 ppm - bezogen auf die Fettalkoholpolyglycolether - eingesetzt.

### Dialkylsulfate

Vorzugsweise setzt man die Fettalkoholpolyglycolether **(II)** und das Dimethylsulfat im molaren Verhältnis von 1 : 1,0 bis 1 : 1,5 und insbesondere 1 : 1,1 bis 1 : 1,4 ein.

### Veretherung

Die Veretherung wird vorteilhafterweise bei einer niedrigeren Temperatur als die Alkoholatbildung durchgeführt. Als optimal hat sich ein Bereich von 20 bis 100 und insbesondere von 40 bis 50°C erwiesen.

### Phasentrennung und Nachbehandlung

Mit der Nachbehandlung der rohen Alkylierungsprodukte mit Wasser werden zwei Ziele verbunden: zum einen wandert die bei der Veretherung gebildete Menge anorganischen Salzes in die Wasserphase, zum anderen wird der Anteil an nichtumgesetzten Dimethylsulfat zersetzt. Hierzu hat es sich als vorteilhaft erwiesen, die Phasentrennung bei einer Temperatur im Bereich von 70 bis 98°C durchzuführen. Nach der Phasentrennung wird der Ether üblicherweise getrocknet und nichtumgesetzte Base abfiltriert. Falls erforderlich, kann der Gehalt an freiem Dimethylsulfat weiter herabgesetzt werden, indem man dem Ether, gegebenenfalls auch schon vor der Phasentrennung, 0,1 bis 1 Gew.-% einer Aminoverbindung wie beispielsweise Ammoniak, Glycin oder ein Alkanolamin zusetzt.

### Gewerbliche Anwendbarkeit

Die nach dem erfindungsgemäßen Verfahren erhältlichen endgruppenverschlossenen Niotenside zeichnen sich durch ein ausgezeichnetes Netzvermögen aus, sind extrem schaumarm und können insbesondere aniontensidhaltige Formulierungen entschäumen. Demzufolge eignen sie sich insbesondere für die Herstellung von Mitteln für die maschinelle Flaschenreinigung, in denen sie in Mengen von 1 bis 50, vorzugsweise 5 bis 35 Gew.-% - bezogen auf die Mittel - enthalten sein können.

Die folgenden Beispiele sollen den Gegenstand der Erfindung näher erläutern, ohne ihn darauf einzuschränken.

### Beispiele

### Beispiele 1 bis 4

In einem Dreihalskolben mit Tropftrichter, Rührer und Rückflußkühler wurden 500 g C₁₂/₁₄- Kokosalkohol+6EO und 500 ppm Natriumborhydrid - bezogen auf den Polyglycolether vorgelegt und unter intensivem Rühren bei einer Temperatur von 80°C über einen Zeitraum von 2 h portionsweise mit Alkalihydroxidschuppen vermischt. Anschließend wurde die Alkoholatmischung auf 45°C abgekühlt, mit Dimethylsulfat versetzt und zunächst 1 h bei 45°C, 2 h bei 50°C und schließlich 1 h bei 60°C gerührt. Danach wurden 500 g Wasser zugegeben, die Mischung auf 80 bis 85°C erwärmt und weitere 2 h gerührt, wobei eine Entmischung stattfand, bei der das gebildete Sulfatsalz praktisch vollständig in der wäßrigen Phase gelöst wurde. Nach der Phasentrennung wurde die organische Wertstoffphase im Vakuum getrocknet und anschließend filtriert. Die Einzelheiten zu den Einsatzverhältnissen und die Kenndaten der Produkte sind in Tabelle 1 zusammengestellt. Die Prozentangaben verstehen sich als Gew.-%.

### Beispiel 5

Beispiel 1 wurde unter Einsatz von Octanol+1PO+10EO wiederholt. Die Ergebnisse befinden sich in Tabelle 1.

### Beispiel 6

Beispiel 1 wurde unter Einsatz von C_{12/14}-Kokosfettalkohol+ 10EO+1PO wiederholt. Die Ergebnisse befinden sich in Tabelle 1.

### Vergleichsbeispiel V1

Beispiel 1 wurde wiederholt, jedoch auf den Zusatz von Natriumborhydrid in der Alkoholatbildung verzichtet. Die Ergebnisse befinden sich in Tabelle 1.

### Vergleichsbeispiel V2

Beispiel 1 wurde wiederholt, anstelle der KOH-Schuppen jedoch eine entsprechende Menge 50 Gew.-%ige Kaliumhydroxidlösung eingesetzt und auf den Zusatz von Natriumborhydrid verzichtet. Die Ergebnisse befinden sich in Tabelle 1.

**Tabelle 1**

| Veretherung mit Dimethylsulfat | | | | | | |
|---|---|---|---|---|---|---|
| **Bsp.** | **Base** | **F:Base** | **F:DMS** | **Ausbeute % d.Th.** | **Salz %** | **Farbe Gard.** |
| 1 | KOH | 1:1,27 | 1:1,15 | 95 | < 1 | < 1 |
| 2 | KOH | 1:1,30 | 1:1,20 | 95 | < 1 | < 1 |
| 3 | KOH | 1:1,40 | 1:1,30 | 94 | < 1 | < 1 |
| 4 | NaOH | 1:1,30 | 1:1,20 | 93 | < 1 | < 1 |
| 5 | KOH | 1:1,27 | 1:1,15 | 95 | < | < 1 |
| 6 | KOH | 1:1,27 | 1:1,15 | 91 | < 1 | < 1 |
| V1 | KOH | 1:1,27 | 1:1,15 | 95 | < 1 | 11 |
| V2 | KOH | 1:1,27 | 1:1,15 | 76 | < 1 | rot |
| Legende: F:Base = Molares Einsatzverhältnis Fettalkohol-polyglycolether : Base F:DMS = Molares Einsatzverhältnis Fettalkohol-polyglycolether : Dimethylsulfat Salz = Gehalt an anorganischem Sulfat im Produkt Farbe = Gardnerfarbzahl | | | | | | |

## Patentansprüche

1. Verfahren zur Herstellung von endgruppenverschlossenen nichtionischen Tensiden der Formel **(I),** 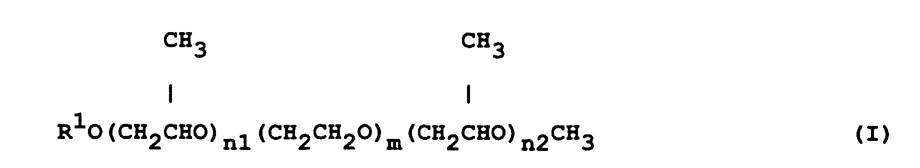 in der R¹ für einen Alkyl- und/oder Alkenylrest mit 6 bis 22 Kohlenstoffatomen, n1 und n2 unabhängig voneinander für 0 oder Zahlen von 1 bis 10 und m für Zahlen von 1 bis 20 steht, durch Umsetzung von Alkoholalkoxylaten mit Dimethylsulfat, **dadurch gekennzeichnet,** daß man
(a) Fettalkoholpolyglycolether der Formel **(II),** 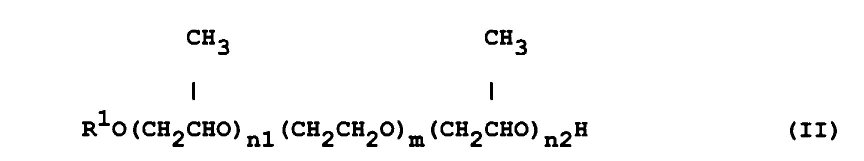 in der R¹, n1, n2 und m die oben angegebenen Bedeutungen besitzen, in Gegenwart von Alkali- und/oder Erdalkaliborhydriden mit festen Basen **(III)** mit einem Wassergehalt von weniger als 15 Gew.-% umsetzt,
(b) die gebildeten Alkoholate mit Dimethylsulfat verethert,
(c) den rohen Ethern eine solche Menge Wasser zusetzt, daß Phasentrennung eintritt und
(d) die organischen Wertphasen in an sich bekannter Weise abtrennt.

2. Verfahren nach Anspruch 1, **dadurch gekennzeichnet,** daß man Fettalkoholpolyglycolether der Formel **(II)** einsetzt, in der R¹ für einen Alkylrest mit 12 bis 18 Kohlenstoffatomen, nl für 0, m für Zahlen von 5 bis 15 und n2 für 1 steht.

3. Verfahren nach Anspruch 1, **dadurch gekennzeichnet,** daß man Fettalkoholpolyglycolether der Formel **(II)** einsetzt, in der R¹ für einen Alkylrest mit 6 bis 10 Kohlenstoffatomen, n1 für 1, m für Zahlen von 5 bis 15 und n2 für 0 steht.

4. Verfahren nach den Ansprüchen 1 bis 3, **dadurch gekennzeichnet,** daß man als Basen Natriumhydroxid und/oder Kaliumhydroxid einsetzt.

5. Verfahren nach den Ansprüchen 1 bis 4, **dadurch gekennzeichnet,** daß man die Fettalkoholpolyglycolether **(II)** und die Basen **(III)** im molaren Einsatzverhältnis von 1 : 1,0 bis 1 : 1,5 einsetzt.

6. Verfahren nach den Ansprüchen 1 bis 5, **dadurch gekennzeichnet,** daß man die Alkoholatbildung bei einer Temperatur im Bereich von 20 bis 98°C durchführt.

7. Verfahren nach den Ansprüchen 1 bis 6, **dadurch gekennzeichnet,** daß man die Borhydride in Mengen von 0,1 bis 1 Gew.-% - bezogen auf die Fettalkoholpolyglycolether - einsetzt.

8. Verfahren nach den Ansprüchen 1 bis 7**, dadurch gekennzeichnet,** daß man die Fettalkoholpolyglycolether **(II)** und das Dimethylsulfat im molaren Verhältnis von 1 : 1,0 bis 1 : 1,5 einsetzt.

9. Verfahren nach den Ansprüchen 1 bis 8, **dadurch gekennzeichnet,** daß man die Veretherung bei einer Temperatur im Bereich von 20 bis 60 °C durchführt.

10. Verfahren nach den Ansprüchen 1 bis 9, **dadurch gekennzeichnet,** daß man die Phasentrennung bei einer Temperatur im Bereich von 70 bis 98 °C durchführt.

## Claims

1. A process for the production of end-capped nonionic surfactants corresponding to formula **(I):** 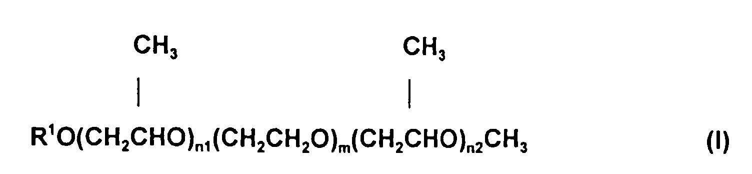 in which R¹ is an alkyl and/or alkenyl group containing 6 to 22 carbon atoms, n1 and n2 independently of one another stand for 0 or for numbers of 1 to 10 and m stands for numbers of 1 to 20,
by reaction of alcohol alkoxylates with dimethyl sulfates, characterized in that
(a) fatty alcohol polyglycol ethers corresponding to formula **(II):** 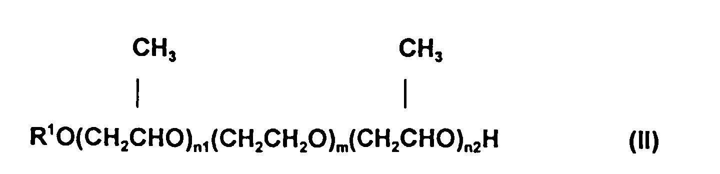 in which R¹, n1, n2 and m are as defined above, are reacted with solid bases (III) having a water content of less than 15% by weight in the presence of alkali metal and/or alkaline earth metal borohydrides,
(b) the alcoholates formed are etherified with dimethyl sulfate,
(c) water is added to the crude ethers in such a quantity that phase separation occurs and
(d) the organic valuable phases are removed by methods known per se.

2. A process as claimed in claim 1, characterized in that fatty alcohol polyglycol ethers corresponding to formula (II), in which R¹ is an alkyl group containing 12 to 18 carbon atoms, n1 stands for 0, m stands for numbers of 5 to 15 and n2 stands for the number 1, are used.

3. A process as claimed in claim 1, characterized in that fatty alcohol polyglycol ethers corresponding to formula (II), in which R¹ is an alkyl group containing 6 to 10 carbon atoms, n1 stands for the number 1, m stands for numbers of 5 to 15 and n2 stands for 0, are used.

4. A process as claimed in claims 1 to 3, characterized in that sodium hydroxide and/or potassium hydroxide is/are used as bases.

5. A process as claimed in claims 1 to 4, characterized in that the fatty alcohol polyglycol ethers (II) and the bases (III) are used in a molar ratio of 1:1.0 to 1:1.5.

6. A process as claimed in claims 1 to 5, characterized in that the alcoholate formation is carried out at a temperature of 20 to 98°C.

7. A process as claimed in claims 1 to 6, characterized in that the borohydrides are used in quantities of 0.1 to 1% by weight, based on the fatty alcohol polyglycol ether.

8. A process as claimed in claims 1 to 7, characterized in that the fatty alcohol polyglycol ethers (II) and the dimethyl sulfate are used in a molar ratio of 1:1.0 to 1:1.5.

9. A process as claimed in claims 1 to 8, characterized in that the etherification is carried out at a temperature of 20 to 60°C.

10. A process as claimed in claims 1 to 9, characterized in that the phase separation is carried out at a temperature of 70 to 98°C.

## Revendications

1. Procédé de fabrication d'agents tensioactifs non ioniques à groupes terminaux fermés de formule (I) 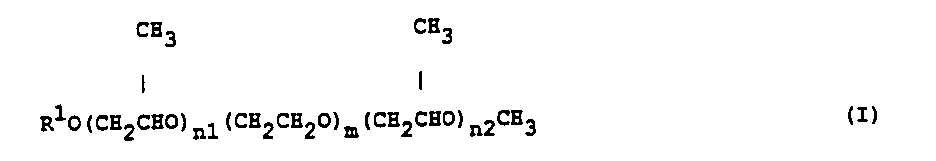 dans laquelle
R¹ représente un radical alkyle et/ou alkényle ayant de 6 à 22 atomes de carbone, n1 et n2 indépendamment l'un de l'autre représentent 0 ou des nombres allant de 1 à 10, et
m représente des nombres allant de 1 à 20,
par réaction d'alcoxylates d'alcool avec le sulfate de diméthyle,
caractérisé en ce que :
a) on fait réagir des éthers d'alcool gras et de polyglycol de formule (II) 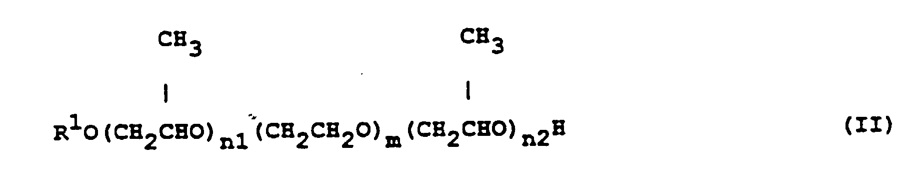 dans laquelle
R¹, n1, n2 et m possèdent les significations mentionnées ci-dessus, en présence de borohydrures de métal alcalin ou alcalino-terreux, avec des bases solides (III) ayant une teneur en eau de moins de 15 % en poids.
b) on éthérifie l'alcoolate formé avec du sulfate de diméthyle,
c) on ajoute aux éthers bruts une quantité d'eau telle qu'une séparation de phase se produise et,
d) on sépare les phases de valeur organiques d'une manière connue en soi.

2. Procédé selon la revendication 1,
caractérisé en ce qu'
on met en oeuvre un éther de polyglycol et d'acide gras de formule (II) dans laquelle R¹ représente un radical alkyle ayant de 12 à 18 atomes de carbone, n1 représente 0, m représente des nombres allant de 5 à 15 et n2 représente 1.

3. Procédé selon la revendication 1,
caractérisé en ce qu'
on met en oeuvre un éther de polyglycol et d'acide gras de formule (II) dans laquelle R¹ représente un radical alkyle ayant de 6 à 10 atomes de carbone, n1 représente 1, m représente des nombres allant de 5 à 15 et n2 représente 0.

4. Procédé selon les revendications 1 à 3,
caractérisé en ce qu'
on met en oeuvre comme bases de l'hydroxyde de sodium et/ou de l'hydroxyde de potassium.

5. Procédé selon les revendications 1 à 4,
caractérisé en ce qu'
on met en oeuvre l'éther de polyglycol et d'alcool gras et les bases (III) dans un rapport d'utilisation molaire allant de 1:1,0 à 1:1,5.

6. Procédé selon les revendications 1 à 5,
caractérisé en ce qu'
on effectue la formation d'alcoolate à une température dans la zone de 20 à 98°C.

7. Procédé selon les revendications 1 à 6,
caractérisé en ce qu'
on met en oeuvre les borohydrures en quantités allant de 0,1 à 1 % en poids, rapporté à l'éther de polyglycol et d'alcool gras.

8. Procédé selon les revendications 1 à 7,
caractérisé en ce qu'
on met en oeuvre l'éther de polyglycol et d'alcool gras et le sulfate de diméthyle dans un rapport molaire de 1:1,0 à 1:1,5.

9. Procédé selon les revendications 1 à 8,
caractérisé en ce qu'
on effectue l'éthérification à une température dans la zone de 20 à 60°C.

10. Procédé selon les revendications 1 à 9,
caractérisé en ce qu'
on effectue la séparation de phase à une température dans la zone de 70 à 98°C.
